# FASCICULE DE BREVET EUROPEEN

(11) **EP 0 806 202 B1**
(45) Date de publication et mention de la délivrance du brevet: **30.01.2002**
(21) Numéro de dépôt: 97400990.4
(22) Date de dépôt: 02.05.1997
(51) Int. Cl.: A61K 9/48

(54) **Composition pharmaceutique à base de matrices lipophiles stabilisées pour la libération controlée de principes actifs**
Pharmazeutische Zusammensetzung aus stabilisierten lipophilen Matrizen mit kontrollierter Wirkstofffreisetzung
Controlled release pharmaceutical composition containing stabilized lipophilic matrices

(30) Priorité: 06.05.1996 FR 9605606
(43) Date de publication de la demande: 12.11.1997
(73) Titulaire: LES LABORATOIRES SERVIER, 92200 Neuilly sur Seine (FR)
(72) Inventeur: Fonknechten, Gilles, 45000 Orleans (FR); Genty, Patrick, 45800 Saint Jean de Braye (FR)

(56) Documents cités:
- EP-A- 0 222 614
- WO-A-96/21439

## Description

La présente invention concerne une nouvelle matrice lipophile pour l'obtention de compositions pharmaceutiques permettant la libération contrôlée de principes actifs.

Cette nouvelle composition est constituée essentiellement par une matrice lipophile stabilisée.

L'obtention de compositions pharmaceutiques permettant la libération contrôlée de principes actifs a pour but essentiel d'obtenir chez l'homme une concentration sanguine du principe acif régulière par écrêtement du pic sanguin généralement obtenu avec les formes à libération immédiate et ainsi non seulement d'éviter les effets indésirables dus à ces pics sanguins, mais encore de diminuer le nombre de prises journalières du médicament, ce qui facilite la compliance du patient au traitement.

Parmi les différentes formes à libération prolongée connues, les matrices lipophiles ont été décrites dans la littérature. Toutefois, le principal inconvénient de ces matrices lipophiles est une évolution de la cinétique de libération du principe actif lors du vieillissement. Les études de stabilité des formes à base de matrices lipophiles montrent une accélération de la cinétique de libération du principe actif, impliquant de ce fait une impossibilité d'obtenir une date de péremption déterminée pour la forme pharmaceutique considérée. Cette évolution de la cinétique de dissolution du principe actif s'explique par une modification de l'état inteme de la matrice. Cette transformation de l'excipient gras a lieu à température ambiante et peut s'étendre sur plusieurs mois.

La matrice lipophile selon l'invention, outre le fait qu'elle soit nouvelle, permet de résoudre le problème de stabilité rencontré avec les autres matrices décritent dans l'Art Antérieur. Elle associe de façon originale deux types de familles chimiques différentes permettant d'obtenir une stabilité complète de la forme pharmaceutique pendant toute la durée de conservation du médicament, ainsi qu'une libération parfaitement contrôlée du principe actif.

Parmi l'art antérieur décrivant des compositions pharmaceutiques de formulation proche, mais dont le but est de résoudre des problèmes techniques autres que celui exposé dans la présente invention, on peut citer à titre indicatif les demandes de brevet EP 0222614 et WO 96/21439.

La première famille de composés est constituée par des mono ou di ou triglycérides d'acides gras ou par des mono ou di ou triglycérides polyglycolysés saturés obtenus à partir d'huiles végétales hydrogénées et constitués de glycérides et d'esters de polyéthylène glycol. Une partie de cette famille est appelé Gélucire®. Cette famille d'excipients est habituellement utilisée pour la réalisation de matrices lipophiles. Ces excipients sont caractérisés par leur point de fusion et par leur valeur HLB (valeur de la balance hydrophile-lipophile). Les excipients utilisés dans la présente invention sont ceux pour lesquels le pouvoir lipophile est important, c'est-à-dire ceux pour lesquels la valeur HLB est inférieure à 10.

La deuxième famille chimique qui permet la stabilisation de la cinétique de dissolution, est constituée d'éthers de cellulose tels la méthylhydroxypropylcellulose, la méthylcellulose, l'hydroxyméthylcellulose, l'hydroxypropylcellulose. La stabilisation de cette matrice doit, d'autre part, être obtenue en conservant les propriétés lipophiles de la matrice. C'est pourquoi dans les matrices selon l'invention, il a été montré que le pourcentage de dérivé de cellulose permettant de stabiliser la matrice ne doit excéder 15 % de la masse totale de la formulation. Ainsi, cette matrice rendue lipophile par l'utilisation d'un dérivé de triglycérides est stabilisée de manière surprenante par l'adjonction d'un dérivé cellulosique.

La matrice lipophile selon l'invention est donc composée de la façon suivante :
- un ou plusieurs dérivés de triglycérides d'acide gras ou polyglycolysé (substances grasses),
- un ou plusieurs dérivés cellulosiques (stabilisants),
- un ou plusieurs principes actifs,
- et, éventuellement, un ou plusieurs excipients pouvant jouer le rôle de plastifiants, d'aromatisants ou d'édulcorants.

La matrice lipophile selon l'invention pourra être avantageusement utilisée pour l'obtention de microsphères ou de microgranules qui seront utilisés pour la fabrication de gélules, de comprimés, d'implants, de gels, de crèmes, de systèmes transdermiques ou transmuqueux, de solutions buvables ou injectables. Une forme préférentielle est l'obtention de gélules.

Parmi les principes actifs utilisés dans la composition pharmaceutique selon l'invention, on peut citer, à titre non limitatif : les antiinfectieux comme les pénicillines, les céphalosporines, les cyclines, les inhibiteurs de béta-lactamases, les aminosides, les quinolones, les nitroimidazolés, les sulfamides ou les antibactériens, les antihistaminiques, les antiallergiques, les anesthésiques, les antiinflammatoires stéroïdiens ou non, les antalgiques d'action locale ou systémique, les antispasmodiques, les anticancéreux, les diurétiques, les béta-bloquants, les antihypertenseurs, les antiangoreux, les antiarythmiques, les vasodilatateurs, les bradycardisants, les inhibiteurs calciques, les sédatifs, les cardiotoniques, les antifungiques, les antiulcéreux, les veinotoniques, les vasculoprotecteurs, les antiischémiques, les antiémétiques, les anticoagulants, les antithrombotiques, les immunosuppresseurs, les immunomodulateurs, les antiviraux, les antidiabétiques, les hypolipidémiants, les antiobésités, les anticonvulsivants, les hypnotiques, les antiparkinsoniens, les antimigraineux, les neuroleptiques, les anxiolytiques, les antidépresseurs, les psychostimulants, les promnésiants, les bronchodilatateurs, les antitussifs, les antistéoporotiques, les hormones peptidiques, les stéroïdes, les enzymes, les inhibiteurs d'enzymes, les agonistes ou antagonistes mélatoninergiques.

Les résultats obtenus avec ces matrices lipophiles montrent que :
- d'une part, il n'y a pas transformation de l'excipient gras en fonction de la température (traitement thermique),
- d'autre part, que cette stabilisation est durable. Aucune modification n'a été observée après plusieurs mois de conservation dans les conditions de conservation standards définies par les normes internationales.
Ainsi, aucun traitement n'est nécessaire pour obtenir une matrice stable, ce qui présente un avantage essentiel au niveau industriel et, par la même, une diminution très importante des coûts de production.

Les matrices lipophiles selon l'invention sont obtenues selon des techniques qui mettent en jeu la fusion, la nébulisation par air chaud ou froid ("spray drying" ou "spray congeling"), l'extrusion à chaud ou à froid, la sphéronisation, ou bien encore, la technique qui consiste à éjecter un liquide à travers une buse munie de nombreux orifices sur laquelle est appliquée une vibration ("prilling").
Un procédé préférentiel pour l'obtention de gélules est le suivant :
***Stade 1 :*** Fusion de la ou des substance(s) grasse(s) dans un fondoir.
***Stade 2 :*** Incorporation d'un ou des éther(s) de cellulose dans la masse fondue de la ou des substance(s) grasses(s). Puis, homogénéisation du mélange à l'aide d'un système approprié.
***Stade 3 :*** Incorporation du principe actif et des autres excipients si il y a lieu.
   Puis, homogénéisation du mélange à l'aide d'un système approprié.
***Stade 4 :*** Remplissage des gélules par le mélange obtenu au stade 3, maintenu en température et homogénéisé en continu afin de garantir une parfaite reproductibilité de la forme pharmaceutique.

Les gélules ainsi obtenues sont éventuellemnt soumises à un traitement thermique.

Les exemples suitants illustrent l'invention, mais ne la limitent en aucune façon.

### EXEMPLE 1 :

### - Sans adjonction du composé stabilisant

Une gélule à libération prolongée est préparée en utilisant la formule donnée dans le tableau qui suit, suivant le procédé de fabrication décrit dans les stades 1, 3 et 4 précédents sans addition d'hydroxypropylméthylcellulose (HPMC), le stabilisant.

| **Nom des constituants** | **Quantités(mg)** |
|---|---|
| Indapamide | 5 |
| Gélucire® 50-02 | 295 |
| Gélule terminée à | 300 |

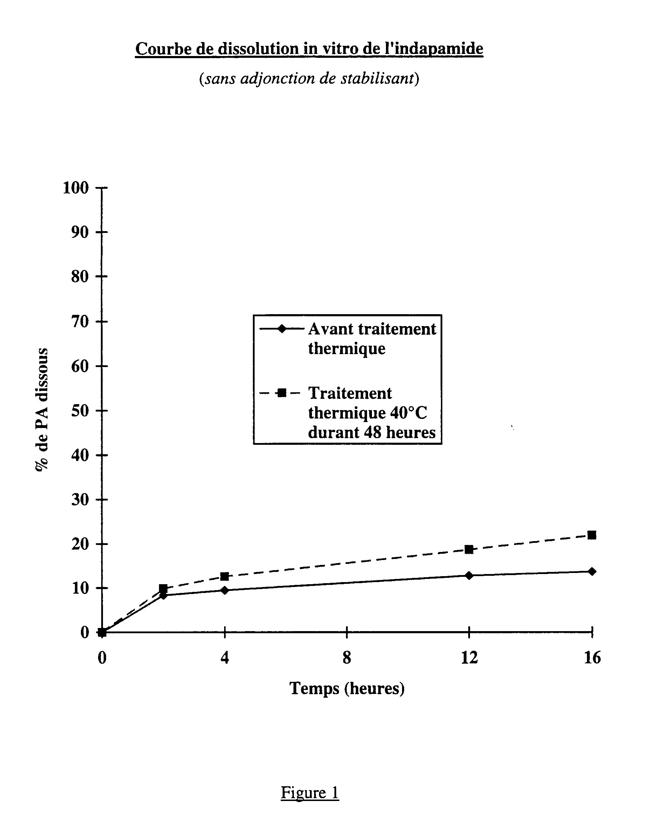

Les courbes de dissolution in vitro avant et après traitement thermique sont présentées en annexe dans la figure 1. On observe une augmentation de la cinétique de libération du principe actif lors du traitement thermique.

### - Avec adjonction du composé stabilisant

Une gélule à libération prolongée est préparée en utilisant une formule identique à la précédente mais avec addition du stabilisant, HPMC. La formule exacte est donnée dans le tableau ci-dessous. Le procédé de fabrication est celui décrit dans les stades 1 à 4 précédents.

| **Nom des constituants** | **Quantités(mg)** |
|---|---|
| Indapamide | 5 |
| Gélucire® 50/02 | 295 |
| Hydroxypropylméthylcellulose | 30 |
| Gélule terminée à | 330 |

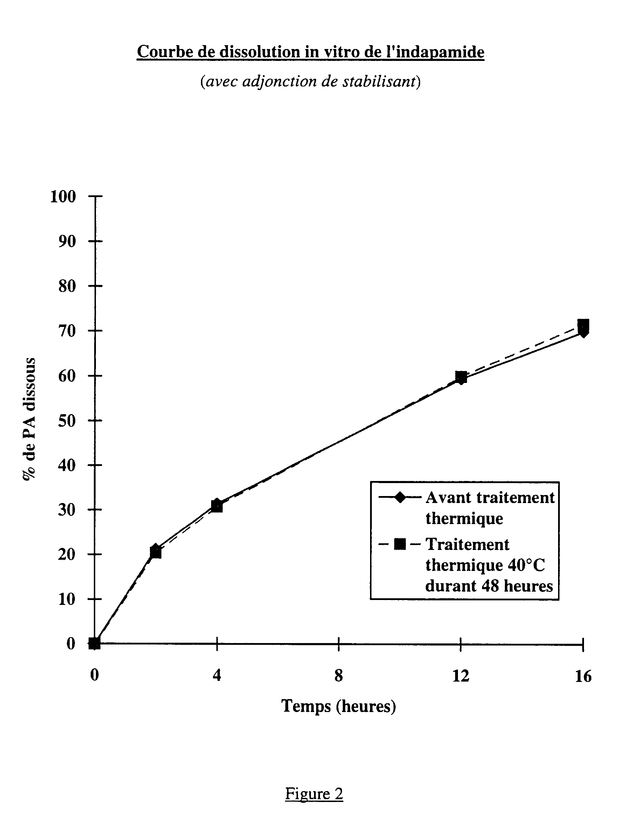

Les courbes de dissolution in vitro avant et après traitement thermique sont présentées en annexe dans la figure 2. On n'observe aucune modification de la cinétique de libération du principe actif lors du traitement thermique.

### EXEMPLE 2:

### - Sans adjonction du composé stabilisant

Une gélule à libération prolongée est préparée en utilisant la formule donnée dans le tableau qui suit, suivant le procédé de fabrication décrit dans les stades 1, 3 et 4 précédents sans addition d'hydroxypropylméthylcellulose (HPMC), le stabilisant.

| **Nom des constituants** | **Quantités(mg)** |
|---|---|
| Fenspiride | 200 |
| Gélucire® 63-03 | 47 |
| Gélucire® 63-05 | 188 |
| Gélule terminée à | 435 |

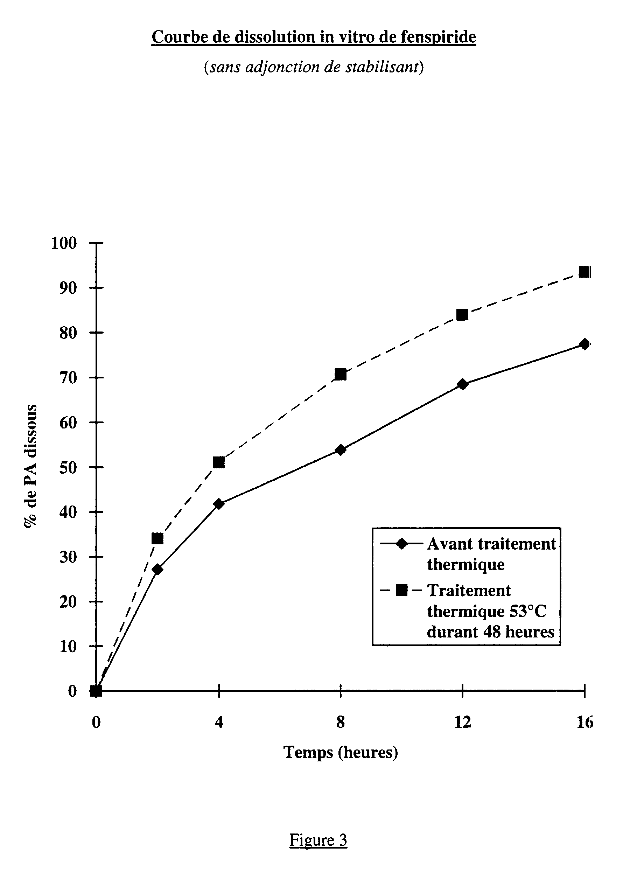

Les courbes de dissolution in vitro avant et après traitement thermique sont présentées en annexe dans la figure 3. On observe une augmentation de la cinétique de libération du principe actif lors du traitement thermique.

### - Avec adjonction du composé stabilisant

Une gélule à libération prolongée est préparée en utilisant une formule identique à la précédente mais avec addition du stabilisant, HPMC. La formule exacte est donnée dans le tableau ci-dessous. Le procédé de fabrication est celui décrit dans les stades 1 à 4 précédents.

| **Nom des constituants** | **Quantités(mg)** |
|---|---|
| Fenspiride | 200 |
| Gélucire® 63-03 | 47 |
| Gélucire® 63-05 | 188 |
| Hydroxypropylméthylcellulose | 45 |
| Gélule terminée à | 480 |

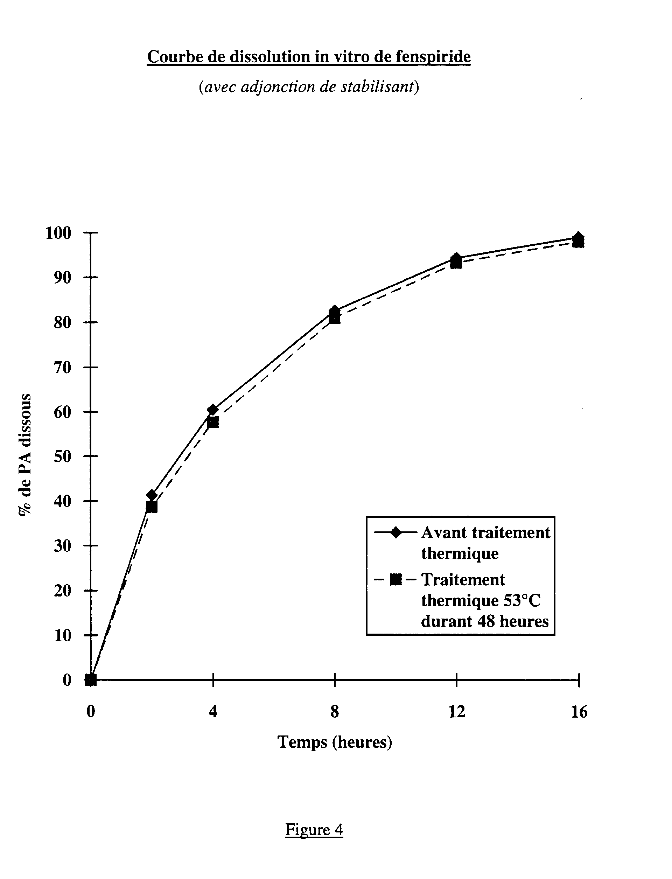

Les courbes de dissolution in vitro avant et après traitement thermique sont présentées en annexe dans la figure 4. On n'observe aucune modification de la cinétique de libération du principe actif lors du traitement thermique.

### EXEMPLE 3 :

| **Nom des constituants** | **Quantités(mg)** |
|---|---|
| Gliclazide | 80 |
| Gélucire® 50-02 | 220 |
| Hydroxypropylméthylcellulose | 30 |
| Gélule terminée à | 330 |

### EXEMPLE 4 :

| **Nom des constituants** | **Quantités(mg)** |
|---|---|
| Tianeptine | 50 |
| Akoline MCM ® | 20 |
| Akomed R ® | 180 |
| Hydroxypropylméthylcellulose | 15 |
| Gélule terminée à | 245 |

### EXEMPLE 5 :

| **Nom des constituants** | **Quantités(mg)** |
|---|---|
| Amineptine | 200 |
| Gélucire® 50-02 | 200 |
| Hydroxypropylméthylcellulose | 40 |
| Gélule terminée à | 440 |

### EXEMPLE 6:

| **Nom des constituants** | **Quantités(mg)** |
|---|---|
| Dexfenfluramine chlorhydrate | 30 |
| Gélucire® 50-02 | 120 |
| Hydroxypropylméthylcellulose | 15 |
| Gélule terminée à | 165 |

### EXEMPLE 7 : Etude de la stabilité des matrices dans les conditions de conservation définies par les normes internationales ICH (International Conference on Harmonisation).

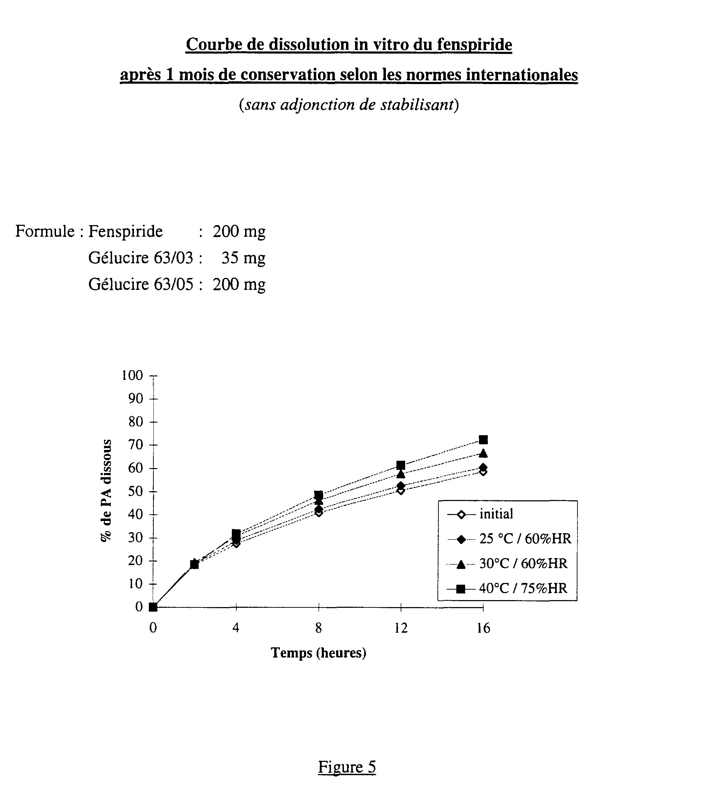

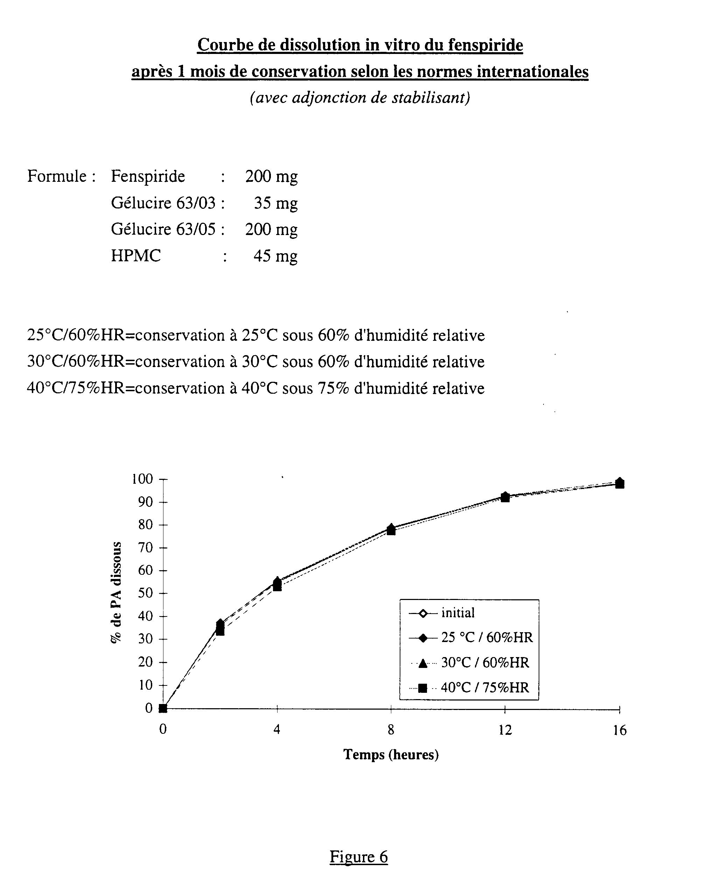

Les formulations utilisées pour cette étude sont celles décrites dans l'exemple 2. Les courbes de dissolution in vitro des gélules contenant ou non de l'hydroxypropylméthylcellulose (HPMC), obtenues après un mois de conservation dans trois conditions différentes, sont présentées en annexe dans les figures 5 et 6. Elles montrent que sans HPMC la matrice est instable, alors que, lorsque ces matrices contiennent de l'HPMC, elles sont stables pendant un mois dans les trois conditions de température et d'humidité relative testées.

### EXEMPLE 8 : Etude de stabilité à long terme des matrices selon l'invention

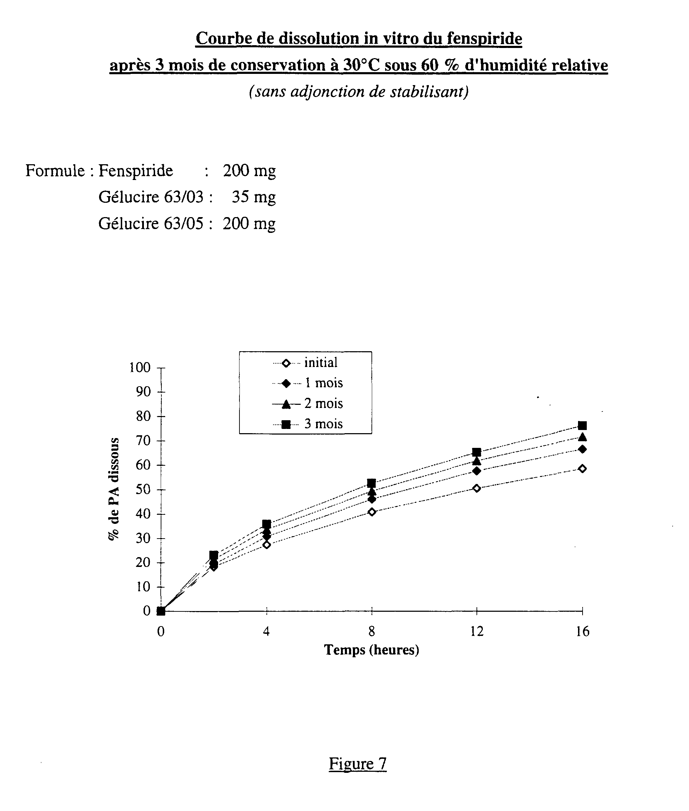

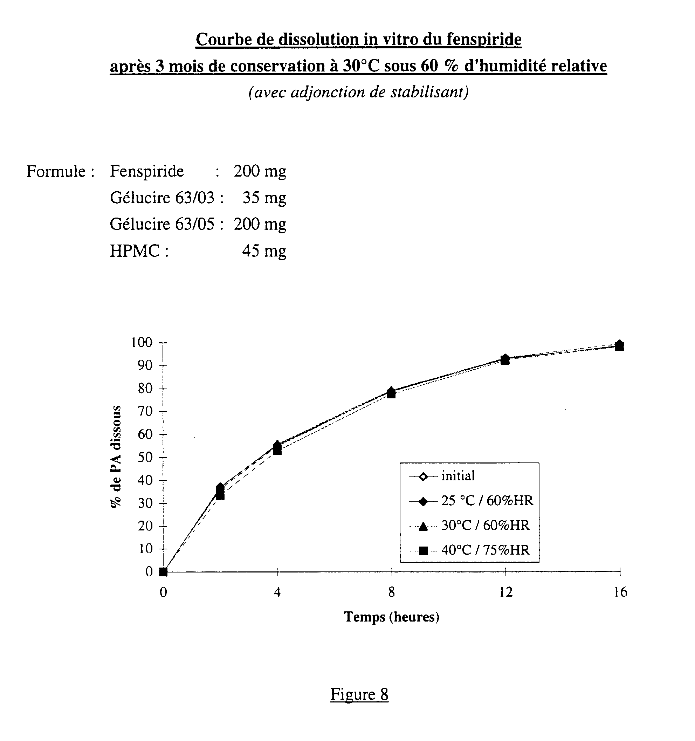

Les formulations utilisées sont celles décrites dans l'exemple 2. Les courbes de dissolution in vitro des gélules testées, obtenues après trois mois de conservation à 30°C sous 60 % d'humidité relative sont présentées en annexe dans les figures 7 et 8. Ces courbes montrent qu'au bout de trois mois les gélules contenant des matrices selon l'invention sont parfaitement stables, alors que celles ne contenant pas d'HPMC présentent une forte instabilité et sont donc inutilisables et impropres à la commercialisation.

## Revendications

1. Matrice lipophile stabilisée pour la libération contrôlée de principes actifs dont le pouvoir lipophile est fourni par l'utilisation de substances grasses dérivées de triglycérides d'acides gras ou polyglycolysés **caractérisée en ce qu'**elle est stabilisée par l'utilisation de dérivés de la cellulose dans une proportion n'excédant pas 15 % de la masse totale de la formulation et que lesdites substances grasses présentent un coefficient HLB inférieur à 10..

2. Matrice lipophile stabilisée selon la revendication 1 **caractérisée en ce que** les substances grasses consistent en un ou plusieurs mono, di ou triglycérides polyglycolysés saturés obtenus à partir d'huiles végétales hydrogénées et constitués de glycérides d'esters de polyéthylène glycols.

3. Matrice lipophile stabilisée selon la revendication 1 **caractérisée en ce que** le dérivé de cellulose utilisé est de l'hydroxypropylméthylcellulose.

4. Matrice lipophile stabilisée selon l'une quelconque des revendications 1 à 3 **caractérisée en ce qu'**elle contient un ou plusieurs principes actifs et excipients pharmaceutiquement acceptables.

5. Matrice lipophile stabilisée selon la revendication 4 **caractérisée en ce que** le ou les principes actifs sont choisis parmi les antiinfectieux comme les pénicillines, les céphalosporines, les cyclines, les inhibiteurs de béta-lactamases, les aminosides, les quinolones, les nitroimidazolés, les sulfamides ou les antibactériens, les antihistaminiques, les antiallergiques, les anesthésiques, les antiinflammatoires stéroïdiens ou non, les antalgiques d'action locale ou systémique, les antispasmodiques, les anticancéreux, les diurétiques, les béta-bloquants, les antihypertenseurs, les antiangoreux, les antiarythmiques, les vasodilatateurs, les bradycardisants, les inhibiteurs calciques, les sédatifs, les cardiotoniques, les antifungiques, les antiulcéreux, les veinotoniques, les vasculoprotecteurs, les antiischémiques, les antiémétiques, les anticoagulants, les antithrombotiques, les immunosuppresseurs, les immunomodulateurs, les antiviraux, les antidiabétiques, les hypolipidémiants, les antiobésités, les anticonvulsivants, les hypnotiques, les antiparkinsoniens, les antimigraineux, les neuroleptiques, les anxiolytiques, les antidépresseurs, les psychostimulants, les promnésiants, les bronchodilatateurs, les antitussifs, les antistéoporotiques, les hormones peptidiques, les stéroïdes, les enzymes, les inhibiteurs d'enzymes, les agonistes ou antagonistes mélatoninergiques.

6. Composition pharmaceutique contenant une matrice lipophile selon l'une quelconque des revendications 1 à 5.

7. Composition pharmaceutique selon la revendication 6 **caractérisée en ce qu'**elle se présente sous la forme de gélules.

8. Procédé d'obtention de la matrice lipophile selon l'une quelconque des revendications 1 à 5 **caractérisée par** les étapes suivantes :
Stade A : fusion de la ou des substances grasses dans un fondoir,
Stade B : incorporation du stabilisant dans la masse fondue obtenue au stade précédent puis homogénéisation,
Stade C : incorporation du ou des principes actifs et des excipients puis homogénéisation.

9. Procédé d'obtention de la composition pharmaceutique selon la revendication 7 **caractérisé en ce que** les gélules sont remplies à l'aide de la matrice lipophile stabilisée obtenue selon la revendication 8, en maintenant la température et en homogénéisant afin d'obtenir une parfaite reproductibilité de la composition pharmaceutique.

## Patentansprüche

1. Stabilisierte lipophile Matrix für die gesteuerte Freisetzung von Wirkstoffen, deren lipophiles Verhalten durch die Verwendung von Fettsubstanzen verursacht wird, die von Triglyceriden von Fettsäuren oder polyglykolisierten Triglyceriden abgeleitet sind, **dadurch gekennzeichnet, daß** sie stabilisiert ist durch die Verwendung von Cellulosederivaten in einem Anteil, der 15 % der Gesamtmasse der Formulierung nicht übersteigt, und daß die Fettsubstanzen einen HLB-Koeffizienten von unterhalb 10 aufweisen.

2. Stabilisierte lipophile Matrix nach Anspruch 1, **dadurch gekennzeichnet, daß** die Fettsubstanzen aus ein oder mehreren gesättigten, polyglykolisierten Mono-, Di- oder Triglyceriden bestehen, welche ausgehend von hydrierten pflanzlichen Ölen erhalten worden sind und aus Glyceriden von Polyethylenglykolestern gebildet sind.

3. Stabilisierte lipophile Matrix nach Anspruch 1, **dadurch gekennzeichnet, daß** das verwendete Cellulosederivat Hydroxypropylmethylcellulose ist.

4. Stabilisierte lipophile Matrix nach einem der Ansprüche 1 bis 3, **dadurch gekennzeichnet, daß** sie einen oder mehrere Wirkstoffe und pharmazeutisch annehmbare Trägermaterialien enthält.

5. Stabilisierte lipophile Matrix nach Anspruch 4, **dadurch gekennzeichnet, daß** der oder die Wirkstoffe ausgewählt sind aus antiinfektiösen Mitteln, wie Penicillinen, Cephalosporinen, Cyclinen, beta-Lactamase-Inhibitoren, Aminosiden, Chinolonen, Nitroimidazolen, Sulfamiden oder antibakteriellen Mitteln, Antihistaminika, Antiallergika, Anästhetika, steroidalen oder nichtsteroidalen antiinflammatorischen Mitteln, Analgetika mit lokaler oder systemischer Wirkung, Antispasmodika, Antikrebsmitteln, Diuretika, Betablockern, Antihypertensiva, Antiangormitteln, Antiarrhythmika, Vasodilatatoren, bradykardisierrenden Mitteln, Calciuminhibitoren, Sedativa, kardiotonischen Mitteln, antifungistischen Mitteln, Antigeschwürmitteln, venentonischen Mitteln, gefäßschützenden Mitteln, Antiischämika, Antiemetika, Antikoagulantien, Antithrombotika, Immunosuppressiva, Immunomodulatoren, antiviralen Mitteln, Antidiabetika, hypolipidämischen Mitteln, Mitteln gegen die Fettsucht, Antikonvulsiva, Hypnotika, Antiparkinsonmitteln, Antimigränemitteln, Neuroleptika, Anxiolytika, Antidepressiva, Psychostimulantien, die Gedächtnisleistung verbessernden Mitteln, Bronchodilatatoren, Antitussiva, Antisteoporotika, Peptidhormonen, Steroiden, Enzymen, Enzyminhibitoren und melatoninergischen Agonisten oder Antagonisten.

6. Pharmazeutische Zubereitung enthaltend eine lipophile Matrix nach einem der Ansprüche 1 bis 5.

7. Pharmazeutische Zubereitung nach Anspruch 6, **dadurch gekennzeichnet, daß** sie in Form von Gelkapseln vorliegt.

8. Verfahren zur Herstellung der lipophilen Matrix nach einem der Ansprüche 1 bis 5, **gekennzeichnet durch** die folgenden Stufen:
Stufe A: Schmelzen des oder der Fettsubstanz(en) in einer Schmelzeinrichtung,
Stufe B: Einarbeiten des Stabilisierungsmittels in die in der vorhergehenden Stufe erhaltene geschmolzene Masse und Homogenisieren,
Stufe C: Einarbeiten des oder der Wirkstoffe und der Trägermaterialien und Homogenisieren.

9. Verfahren zur Herstellung der pharmazeutischen Zubereitung nach Anspruch 7, **dadurch gekennzeichnet, daß** die Gelkapseln mit der stabilisierten lipophilen Matrix erhalten nach Anspruch 8 gefüllt werden, wobei man die Temperatur beibehält und homogenisiert, um eine perfekte Reproduzierbarkeit der pharmazeutischen Zubereitung zu erreichen.

## Claims

1. Stabilised lipophilic matrix for the controlled release of active ingredients whose lipophilic power is provided by the use of fatty substances derived from triglycerides of fatty or polyglycolysated acids, **characterised in that** it is stabilised by the use of cellulose derivatives in a proportion not exceeding 15 % of the total weight of the formulation; and the said fatty substances have an HLB coefficient of less than 10.

2. Stabilised lipophilic matrix according to claim 1, **characterised in that** the fatty substances consist of one or more saturated polyglycolysated mono-, di- or triglycerides obtained from hydrogenated vegetable oils and composed of glycerides of esters of polyethylene glycols.

3. Stabilised lipophilic matrix according to claim 1, **characterised in that** the cellulose derivative used is hydroxypropyl methyl cellulose.

4. Stabilised lipophilic matrix according to any one of claims 1 to 3, **characterised in that** it comprises one or more active ingredients and pharmaceutically acceptable excipients.

5. Stabilised lipophilic matrix according to claim 4, **characterised in that** the active ingredient or ingredients is/are selected from anti-infective agents such as penicillins, cephalosporins, cyclines, beta-lactamase inhibitors, aminosides, quinolones, nitroimidazole compounds, sulphamides or antibacterials, antihistamines, anti-allergics, anaesthetics, steroidal or non-steroidal anti-inflammatories, antalgics having local or systemic action, antispasmodics, anti-cancer agents, diuretics, beta-blockers, antihypertensives, anti-angina agents, anti-arrythmics, vasodilators, bradycardiacs, calcium inhibitors, sedatives, cardiotonics, antifungals, anti-ulcerative agents, venotonics, vasculoprotectors, anti-ischaemics, anti-emetics, anticoagulants, antithrombotics, immunosuppressors, immunomodulators, antivirals, antidiabetics, hypolipidaemic agents, anti-obesity agents, anticonvulsants, hypnotics, antiparkinsonian agents, antimigraine agents, neuroleptics, anxiolytics, antidepressants, psychostimulants, memory-enhancers, bronchodilators, antitussives, anti-osteoporotics, peptide hormones, steroids, enzymes, enzyme inhibitors, and melatoninergic agonists and antagonists.

6. Pharmaceutical composition comprising a lipophilic matrix according to any one of claims 1 to 5.

7. Pharmaceutical composition according to claim 6, **characterised in that** it is presented in the form of gelatin capsules.

8. Process for obtaining the lipophilic matrix according to any one of claims 1 to 5, **characterised by** the following steps:
Step A : melting of the fatty substance(s) in a melting vessel,
Step B: incorporation of the stabiliser in the melted mass obtained in the previous Step, and then homogenisation,
Step C: incorporation of the active ingredient(s) and excipients, and then homogenisation.

9. Process for obtaining the pharmaceutical composition according to claim 7, **characterised in that** the gelatin capsules are filled using the stabilised lipophilic matrix obtained according to claim 8, whilst maintaining the temperature and homogenising to obtain perfect reproducibility of the pharmaceutical composition.
